# EUROPEAN PATENT SPECIFICATION

(11) **EP 1 722 821 B1**
(45) Date of publication and mention of the grant of the patent: **13.01.2016**
(21) Application number: 05721856.2
(22) Date of filing: 16.02.2005
(51) Int. Cl.: A61K 47/38, A61K 9/14, A61K 31/18, A61P 13/08, A61K 9/50, A61K 9/16

(54) **COMPOSITION FOR ORAL ADMINISTRATION OF TAMSULOSIN HYDROCHLORIDE AND CONTROLLED RELEASE GRANULE FORMULATION COMPRISING SAME**
ZUSAMMENSETZUNG ZUR ORALEN VERABREICHUNG VON TAMSULOSIN-HYDROCHLORID UND DIESE ENTHALTENDE GRANULATFORMULIERUNG MIT KONTROLLIERTER FREISETZUNG
COMPOSITION DESTINEE A L'ADMINISTRATION ORALE D'HYDROCHLORURE DE TAMSULOSINE ET FORMULATION EN GRANULES A LIBERATION CONTROLEE COMPRENANT CETTE COMPOSITION

(30) Priority: 17.02.2004 KR 2004010384
(43) Date of publication of application: 22.11.2006
(73) Proprietor: Hanmi Pharm. Co., Ltd., Hwaseong-gun, Kyungki-do 445-910 (KR)
(72) Inventor: WOO, Jong Soo, Suwon-si, Kyungki-do 440-300 (KR); CHANG, Hee Chul, Suwon-si, Kyungki-do 441-360 (KR)
(74) Representative: Grünecker Patent- und Rechtsanwälte PartG mbB
(86) International application number: PCT/KR2005/000422
(87) International publication number: WO 2005/077420

(56) References cited:
- WO-A1-2005/004851
- WO-A1-2005/044235
- WO-A2-01/78725
- US-B2- 6 569 463
- US-B2- 6 638 535
- MICHEL ET AL.: 'Efficacy nd safety of tamsulosin in the treatment of urological diseases.' EXPERT OPIN.PHARMACOTHER vol. 5, no. 1, January 2004, pages 151 - 160, XP008110679
- KIRBY ET AL.: 'A randomized, double-blind crossover study of tamsulosin and controlled-release doxazosin in patients with benign prostatic hyperplasia.' BJU INTERNATIONAL. vol. 91, no. 1, January 2003, pages 41 - 44, XP008110683

## Description

### Field of the Invention

The present invention relates to a composition for oral administration of tamsulosin hydrochloride, which exhibits an excellent stability and a sustained release of tamsulosin hydrochloride, and a controlled release granule formulation comprising the same.

### Description of the Prior Art

Tamsulosin hydrochloride are currently available for treating benign prostatic hypertrophy, and there have been made many attempts to develop a controlled release formulation of tamsulosin hydrochloride having good stability and an extended release rate. For example, European Patent Publication No. 80341A discloses an oral formulation for controlled release of tamsulosin which contains multiple drug preparations; and Korean Patent Publication No. 1993-7245 discloses a controlled release formulation for oral use comprising the drug, an aggregate-forming agent such as cellulose, chitin and chitosan, and an insoluble polymer. However the stability and the release characteristics of these formulations fluctuate unsatisfactorily depending on the retained food or pH changes in the gastroenteric organs.

WO 01/78725 A2 relates to a pharmaceutical composition comprising an active agent, which can be tamsulosin hydrochloride. The active agent may be associated with hydroxypropylmethylcellulose and with a release controlling agent such as polyvinylacetate.

The present inventors have endeavored to develop a composition having good stability and sustained release characteristics of tamsulosin hydrochloride under any digestive conditions; and have unexpectedly found that a composition comprising tamsulosin hydrochloride, polyvinyl acetate and a water-soluble hydroxypropylmethylcellulose is particularly suitable for oral administration.

### Summary of the Invention

Accordingly, it is a primary object of the present invention to provide a novel tamsulosin hydrochloride composition which exhibits high stability and satisfactory sustained release characteristics of tamsulosin hydrochloride upon oral administration.

It is another object of the present invention to provide a controlled release granule formulation comprising said composition.

In accordance with one aspect of the present invention, there is provided a composition for oral administration of tamsulosin hydrochloride comprising tamsulosin hydrochloride, polyvinylacetate, and a water-soluble hydroxypropylmethylcellulose.

In accordance with another aspect of the present invention, there is provided a sustained release granule of tamsulosin hydrochloride comprising tamsulosin hydrochloride, polyvinylacetate, a water-soluble hydroxypropylmethylcellulose, and a granulating agent.

### Brief Description of the Drawings

The above and other objects and features of the present invention will become apparent from the following description of the invention, when taken in conjunction with the accompanying drawings, which respectively show:
FIG. 1: Dissolution profiles of tamsulosin hydrochloride observed for the hard capsule prepared in Example 17 and Harnal capsule (Jeil Pharm., Korea);
FIGs. 2A and 2B: Dissolution profiles of tamsulosin hydrochloride observed for the hard capsule prepared in Example 17 (A) and Harmal capsule (B) after storage for 10 days under the condition of Test Example 2; and

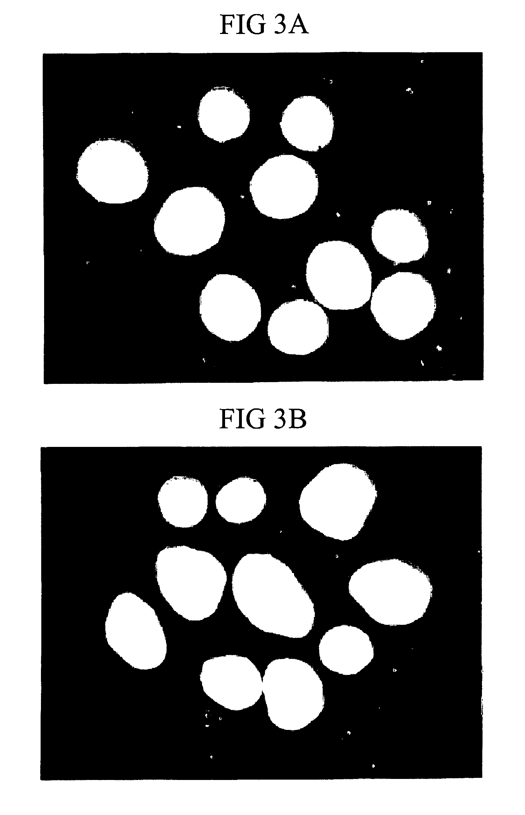

FIGs. 3A and 3B: Microscopic photographs of the coated granule prepared in Example 15 (A) and the granule of Harnal capsule (B).

### Detailed Description of the Invention

The composition of the present invention comprises tamsulosin hydrochloride, polyvinylacetate, and a water-soluble hydroxypropylcellulose.

The inventive composition may further comprise various granulating agents, coating materials, and pharmaceutically acceptable additives.

Each ingredient of the inventive composition and granule formulation is described in detail as follows.

### Tamsulosin hydrochloride

Tamsulosin hydrochloride, the active ingredient of the inventive composition, has a low water-solubility. A typical daily do of tamsulosin hydrochloride in case of treating urination disorder accompanying benign prostatic hypertrophy ranges from 0.2 to 0.8 mg, and it should be administered once a day 30 min before a meal. However, it should be understood that the dosage of tamsulosin hydrochloride should be determined in light of various relevant factors including the condition to be treated, the severity of the patient's symptoms, the route of administration, or the physiological form of the anticancer agent; and therefore, the dosage suggested above should not be construed to limit the scope of the invention in anyway.

### Polyvinylacetate

Polyvinylacetate plays an important role in assisting granulating agent in the process of forming granules and maintaining pores formed in the granules for some period of time after the inventive granule formulation is dissolved in an aqueous medium. Consequently, polyvinylacetate confers on the composition of the inventive composition with sustained release capability of the active ingredient for an extended period of time regardless of pH of the aqueous medium.

In the inventive composition, polyvinylacetate may be used alone or in the form of a mixture with other pharmaceutically acceptable materials, preferably in the form of a powder or a suspension containing more than 30% by weight or more of polyvinylacetate.

For example, Kollidon SR^{®} (BASF), a powder prepared by mixing polyvinylacetate and polyvinylpyrrolidone at a ratio of 8:2 (w/w) and spray-drying the mixture, and Kollicoat SR30D^{®} (solid content: 30%, BASF), a suspension (or a diluted aqueous solution) prepared by mixing polyvinylacetate, polyvinylpyrrolidone and sodium lauryl sulfate, and suspending the mixture in water, may be used in the present invention as a source of polyvinylacetate. In addition, any pharmaceutically acceptable material may be used as a source of polyvinylacetate insofar as it contains 30% by weight or more of polyvinylacetate.

Polyvinylacetate may be employed in the inventive composition in an amount ranging from 20 to 1000 parts by weight, preferably 40 to 600 parts by weight, more preferably 50 to 300 parts by weight based on 1 part by weight of tamsulosin hydrochloride.

### Water-soluble hydroxypropylmethylcellulose

A water-soluble hydroxypropylmethylcellulose (HPMC) controls the initial-phase release of the active ingredient by forming pores, through which the active ingredient is released. In order to obtain a desired sustained release pattern, it is preferable to employ a water-soluble HPMC having a high viscosity, and the desired effect may not be achieved when a low-viscosity water-soluble HPMC is employed. Therefore, the water-soluble HPMC having a high viscosity of more than 10,000 cps, preferably 15,000 to 100,000 cps, is used in the present invention, and representative examples thereof include METOLOSE 60SH, 65SH and 90SH (Shin-Etsu).

The water-soluble HPMC may be used in an amount ranging from 0.1 to 500 parts by weight, preferably 1 to 100 parts by weight, more preferably 2 to 50 parts by weight based on 1 part by weight of tamsulosin hydrochloride.

A controlled release granule formulation prepared by using the inventive composition may further comprise the following ingredients.

### Granulating agent

The inventive granule formulation may comprise a granulating agent and examples thereof include microcrystalline cellulose, lactose and inorganic carrier such as dibasic calcium phosphate, dibasic calcium phosphate dihydrate and tribasic calcium phosphate, wherein microcrystalline cellulose and dibasic calcium phosphate (e.g. A-Tab^{®}, Rhodia) are prefered.

The granulating agent may be used in an amount ranging from 1 to 2000 parts by weight, preferably 10 to 1000 parts by weight based on 1 part by weight of tamsulosin hydrochloride.

### Coating material

The inventive granule formulation of tamsulosin hydrochloride may further be coated with a conventional enteric coating material or a polymeric coating material for the purpose of precisely controlling the absorption of the active ingredient in the gastrointestinal treat.

Examples of the enteric coating material include hydroxypropylmethylcellulose phthalate, hydroxypropylmethylcellulose acetate succinate, polyvinylacetate phthalate, cellulose acetate phthalate, shellac, methacrylate-methylmethacrylate copolymer and methacrylate-ethylacrylate copolymer. Examples of the polymeric coating material include hydroxypropylmethylcellulose, methylcellulose, ethylcellulose, polyvinylacetate and a mixture thereof.

The coating material may be employed in an amount ranging from 0.2 to 100 parts by weight, preferably 1 to 50 parts by weight based on the amount 1 part by weight of tamsulosin hydrochloride.

### Pharmaceutically acceptable additives

The inventive granule formulation may further comprise conventional pharmaceutically acceptable additives for preparing it into various formulations, and exemplary pharmaceutically acceptable additive include a conventional plasticizer, lubricant and other aid.

The pharmaceutical acceptable additive may be employed in an amount ranging from 0.1 to 500 parts by weight, preferably 1 to 200 parts by weight, more preferably 2 to 50 parts by weight based on 1 part by weight of tamsulosin hydrochloride.

The inventive composition of tamsulosin hydrochloride may be formulated into a granule formulation by a conventional method comprising the steps of (i) mixing the composition with a granulating agent, and (ii) subjecting the mixture to wet grinding, compression molding and spheronizing to obtain a wet granule. The granule may be further coated with a coating material dissolved in water to obtain a coated granule. If necessary, the granule may be further mixed with pharmaceutical acceptable additives and filled into a hard gelatin capsule to obtain a capsule formulation.

The following Examples are intended to further illustrate the present invention without limiting its scope.

### I. Preparation of controlled release granule formulation of tamsulosin hydrochloride

### Example 1

0.2 part by weight of tamsulosin hydrochloride, 21.0 parts by weight of Kollicoat SR30D (BASF, polyvinylacetate), 5.5 parts by weight of METOLOSE 90SH (water-soluble HPMC; viscosity: 100,000 cps), and 123.5 parts by weight of micro crystalline cellulose (granulating agent) were placed in a high speed mixer, and an appropriate amount of water was added thereto. The mixture was mixed for 10 to 15 min and treated with a wet grinder obtain a ground material, which was passed through a compression mold equipped with a 0.8 mm mesh, and granulated with a sheronizer to obtain a desired granule.

### Example 2

The procedure of Example 1 was repeated except for using 133.5 parts by weight of dibasic calcium phosphate as a granulating agent, to obtain a desired granule.

### Example 3

The procedure of Example 1 was repeated except for using 95.5 parts by weight of dibasic calcium phosphate dihydrate as a granulating agent, to obtain a desired granule.

### Example 4

The procedure of Example 1 was repeated except for using 128.5 parts by weight of lactose as a granulating agent, to obtain a desired granule.

### Example 5

The procedure of Example 1 was repeated except for using a mixture of 60 parts by weight of lactose and 68.5 parts by weight of microcrystalline cellulose as a granulating agent, to obtain a desired granule.

### Example 6

The procedure of Example 1 was repeated except for using 3.8 parts by weight of METOLOSE 65SH (viscosity: 4,000 cps) instead of METOLOSE 90SH, to obtain a desired granule.

### Example 7

The procedure of Example 1 was repeated except for using 4.5 parts by weight of METOLOSE 65SH instead of METOLOSE 90SH, to obtain a desired granule.

### Example 8

The procedure of Example 1 was repeated except for using 70.0 parts by weight of Kollidon SR instead of Kollicoat SR30D, and 34.5 parts by weight of METOLOSE 65SH instead of METOLOSE 90SH, to obtain a desired granule.

### Example 9

The procedure of Example 1 was repeated except for using 78.0 parts by weight of Kollidon SR instead of Kollicoat SR30D, and 55.5 parts by weight of microcrystalline cellulose instead of 123.5 parts by weight, obtain a desired granule.

### II. Preparation of coated-controlled release granule of tamsulosin hydrochloride

### Example 10

150.0 parts by weight of the controlled release granule of tamsulosin hydrochloride obtained in Example 1 was placed in NQ-160 fluidized bed (DALTON) and bottom-sprayed with a coating solution comprising 9.7 parts by weight of Kollicoat SR30D (solid content: 2.9 parts by weight, a coating material), a mixture of 0.56 part by weight of polyvinylpyrrolidone and 0.43 part by weight of propyleneglycol (a plasticizer), and 18.0 parts by weight of distilled water, to obtain a desired coated granule. During the coating, the entrance temperature was 36 to 39°C, exit temperature was 26 to 28°C, the rate of injection of the coating solution was 0.7 to 0.8 ml /min, and the spraying air pressure was 45 to 55 psi.

### Example 11

The procedure of Example 10 was repeated except for using 3.4 parts by weight of ethylcellulose (IPI) and 7.6 parts by weight of hydroxypropylmethylcellulose (Shin-Etsu) as a coating material, to obtain a desired coated granule.

### Example 12

The procedure of Example 10 was repeated except for using a coating solution comprising 12.0 parts by weight of Eudragit L30D-55 (methacrylate-ethylacrylate copolymer, solid content: 3.6 parts by weight, Roehm) as a coating material, 0.54 part by weight of triacetine as a plasticizer and 21.8 parts by weight of water in place of the coating solution of Example 10, to obtain a desired coated granule.

### Example 13

The procedure of Example 10 was repeated except for using 4.0 parts by weight of hydroxypropylmethylcellulose phthalate (solid content: 3.6 parts by weight, Roehm) as a coating material, to obtain a desired coated granule.

### Example 14

The procedure of Example 10 was repeated except for using 9.0 parts by weight of Eudragit E-100 (methacrylate-methylmethacrylate copolymer, solid content: 3.6 parts by weight, Roehm) as a coating material, to obtain a desired coated granule.

### Example 15

The procedure of Example 10 was repeated except for using 153.9 parts by weight of the coated-granule obrained in Example 10 instead of the granule obtained in Example 1, and a coating solution comprising 12.0 parts by weight of Eudragit L30D-55 (methacrylate-ethylacrylate copolymer, solid content: 3.6 parts by weight, Roehm) as a coating material, 0.54 part by weight of triacetine as a plasticizer and 21.8 parts by weight of water in place of the coating solution of Example 10, to obtain a desired coated granule.

### III. Preparation of hard capsule comprising controlled release granule of tamsulosine hydrochloride

### Example 16

158.14 parts by weight of the coated granule obtained in Example 13, 0.5 part by weight of talc, and 0.5 part by weight of calcium stearate were mixed, and a hard capsule was filled with the mixture to obtain a desired hard capsule.

### Example 17

158.14 parts by weight of the coated granule obtained in Example 15, 0.5 part by weight of talc, and 0.5 part by weight of calcium stearate were mixed, and a hard capsule was filled with the mixture to obtain a desired hard capsule.

### Test Example 1: Dissolution Test

A dissolution test for tamsulosin hydrochloride was conducted using the capsule obtained in Example 17 and Harnal (Jeil Pharm.) capsule as a comparative preparation as follows.

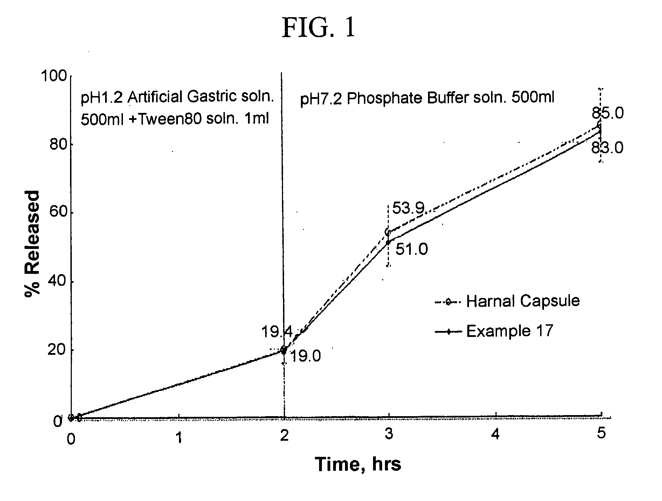

500 ml of artificial gastric juice (pH 1.2) containing 1 ml of Tween 80 was used as test solution (1). Each treat capsule was added to test solution (1), the mixture was agitated at 37±0.5°C and 100 rpm for 2 hours, and a 10 ml sample was taken from the test solution (1). Then, the test solution (1) was changed with test solution (2), 500 ml of a phosphate buffer (pH 7.2), the same agitating procedure was repeated, and 10 ml samples were taken from the test solution (2) at hour 1 and 3 after the start of the agitation, respectively. The sample taken from the test solution (1) was mixed with 2.0 ml of an internal standard (propyl parahydroxybenzoate dissolved in a mixture of water:acetonitrile=7:3), while each of the samples taken from the test solution (2) was mixed with a mixture of 0.5 N HCl and 2.0 ml of the internal standard. The resulting mixture was filtered through a 0.5 µm membrane filter, and subjected to liquid chromatography (column: Cosmosil (ODS) (4.6x150 mm, 5µm) C₁₈; temperature: 40°C; mobile phase: aqueous HClO₄ (adjusted to pH 2.0 using NaOH):acetonitrile=7:3; flow rate: 1.0 ml/min; injection volume: 500 µl; and wave length: 225 nm) to analyze the time-dependent released amount of tamsulosin hydrochloride. The results are shown in FIG. 1.

As shown in FIG. 1, the capsule of the present invention and Harnal capsule exhibited similar tamsulosin hydrochloride release patterns regardless of the pH.

### Test Example 2: Stability Test

12 of the hard capsules obtained in Example 17 and 12 of Harnal capsule (Jeil Pharm.) were each placed into a HDPE bottle. Then, the bottle was sealed, and kept at 60°C, or at 40°C and 75% relative humidity (a stress condition).

At days 0, 10 or 30 of in such treatment, each residual percentage of tamsulosin hydrochloride in the inventive or Harnal capsule was analyzed using a liquid chromatography, and the results is listed in Table I.

**Table I**

| | 0 day | 10 days | 30 days |
|---|---|---|---|
| Example 17 | 100±1.75% | 98.7±1.36% | 93.5±3.98% |
| Harnal | 100±3.43% | 98.6±0.0% | 91.0±3.34% |

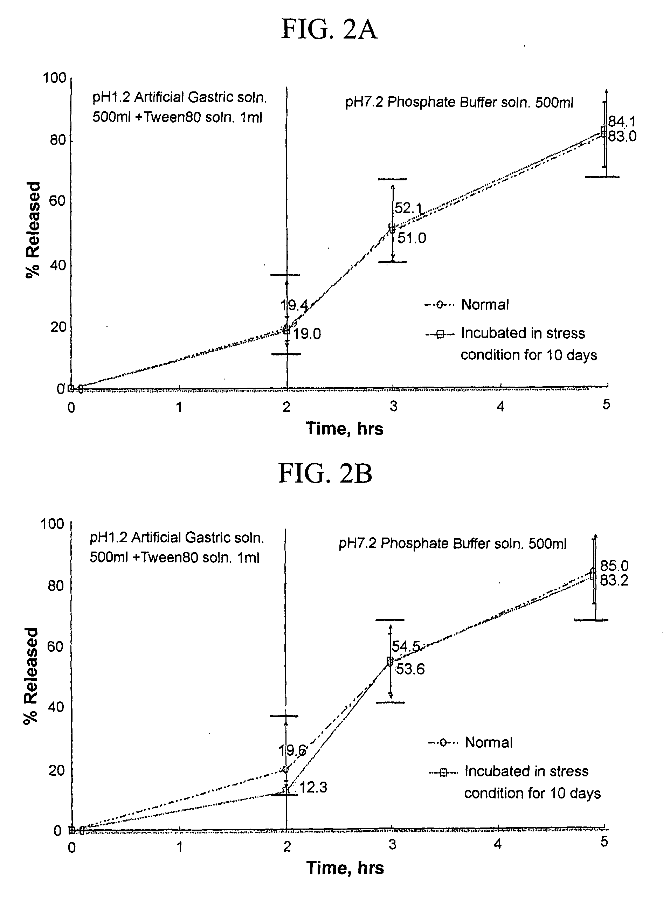

Further, using the inventive and Harnal capsules kepted for 10 days under the above stress condition, the procedure of Test Example 1 was repeated. The results are shown in FIGs. 2A (the inventive capsule) and 2B (Harnal capsule).

As the results show, the inventive capsule of tamsulosin hydrochloride exhibited high stability and good sustained release charateristics of tamsulosin hydrochloride, comparable to Harnal capsule.

### Test Example 3: Sphericity Test

10-granule particles of the coated granule obtained in Example 15 and those of Harnal capsule were each examined using a microscope (Nikkon SMZ800). On each microscopic photograph, a least circumscribed circle of the granule was drawn, and the distance from the circumcenter and surface of the granule was measured to obtain the minimum distance (A) and maximum distance (B). The sphericity of each granule was evaluated by A/B, and the results are listed in Table II.

**Table II**

| Granule No. | 1 | 2 | 3 | 4 | 5 | 6 | 7 | 8 | 9 | 10 | Average | Deviation |
|---|---|---|---|---|---|---|---|---|---|---|---|---|
| Example 15 | 0.96 | 0.93 | 0.89 | 0.89 | 0.91 | 0.86 | 0.87 | 0.83 | 0.88 | 0.83 | 0.89 | 0.04 |
| Harnal | 0.80 | 0.80 | 0.88 | 0.92 | 0.65 | 0.81 | 0.78 | 0.88 | 0.80 | 0.63 | 0.79 | 0.10 |

The results in Table II suggest that the granule of the present invention is more spherical than the granule of Harnal, for the average A/B of the inventive granule was closer to 1 than the comparative granule. Thus, composition of the present invention is capable of forming uniform granules.

As can be seen from the above, the inventive composition for oral administration of tamsulosin hydrochloride having high stability, a good sustained release rate of the active ingredient and the capability for forming uniform granules can be advantageously used in various fields including medical science and pharmaceutical chemistry.

While the invention has been described with respect to the specific embodiments, it should be recognized that various modifications and changes may be made by those skilled in the art to the invention which also fall within the scope of the invention as defined by the appended claims.

## Claims

1. A composition for oral administration of tamsulosin hydrochloride comprising tamsulosin hydrochloride, polyvinylacetate, and a water-soluble hydroxypropylmethylcellulose, wherein the amount of polyvinylacetate ranges from 20 to 1000 parts by weight based on 1 part by weight of tamsulosin hydrochloride.

2. The composition of claim 1, wherein polyvinylacetate is in the form of a powder or suspension comprising polyvinylacetate and a pharmaceutically acceptable additive.

3. The composition of claim 1, wherein the amount of water-soluble hydroxypropylmethylcellulose ranges from 0.1 to 500 parts by weight based on 1 part by weight of tamsulosin hydrochloride.

4. A sustained release granule of tamsulosin hydrochloride comprising tamsulosin hydrochloride, polyvinylacetate, a water-soluble hydroxypropylmethylcellulose, and a granulating agent, wherein the amount of polyvinylacetate ranges from 20 to 1000 parts by weight based on 1 part by weight of tamsulosin hydrochloride.

5. The granule of claim 4, wherein the granulating agent is selected from the group consisting of lactose, microcrystalline cellulose, dibasic calcium phosphate, dibasic calcium phosphate dihydrate, tribasic calcium phosphate and a mixture thereof.

6. The granule of claim 4, wherein the amount of the granulating agent ranges from 1 to 2000 parts by weight based on 1 part by weight of tamsulosin hydrochloride.

7. The granule of claim 4, which is coated with a coating material.

8. The granule of claim 7, wherein the coating material is a polymeric or an enteric coating material.

9. The granule of claim 7, wherein the amount of the coating material ranges from 0.2 to 100 parts by weight based on 1 part by weight of tamsulosin hydrochloride.

10. The composition of claim 1 and the granule of claim 4, wherein the amount of polyvinylacetate ranges from from 40 to 600, preferably from 50-300 parts by weight based on 1 part by weight of tamsulosin hydrochloride, preferably.

## Patentansprüche

1. Zusammensetzung zur oralen Verabreichung von Tamsulosin-hydrochlorid, umfassend Tamsulosin-hydrochlorid, Polyvinylacetat und eine wasserlösliche Hydroxypropylmethylcellulose, wobei die Menge an Polyvinylacetat im Bereich von 20 bis 1000 Gewichtsteilen, bezogen auf 1 Gewichtsteil Tamsulosin-hydrochlorid, liegt.

2. Zusammensetzung nach Anspruch 1, wobei Polyvinylacetat in Form eines Pulvers oder einer Suspension, umfassend Polyvinylacetat und einen pharmazeutisch verträglichen Zusatz, vorliegt.

3. Zusammensetzung nach Anspruch 1, wobei die Menge an wasserlöslicher Hydroxypropylmethylcellulose im Bereich von 0,1 bis 500 Gewichtsteilen, bezogen auf 1 Gewichtsteil Tamsulosin-hydrochlorid, liegt.

4. Granulat mit verzögerter Freisetzung von Tamsulosin-hydrochlorid, umfassend Tamsulosin-hydrochlorid, Polyvinylacetat, eine wasserlösliche Hydroxypropylmethylcellulose und ein Granuliermittel, wobei die Menge an Polyvinylacetat im Bereich von 20 bis 1000 Gewichtsteilen, bezogen auf 1 Gewichtsteil Tamsulosin-hydrochlorid, liegt.

5. Granulat nach Anspruch 4, wobei das Granuliermittel aus der Gruppe bestehend aus Lactose, mikrokristalliner Cellulose, Dicalciumphosphat, Dicalciumphosphat-dihydrat, Tricalciumphosphat und einer Mischung davon ausgewählt ist.

6. Granulat nach Anspruch 4, wobei die Menge des Granuliermittels im Bereich von 1 bis 2000 Gewichtsteilen, bezogen auf 1 Gewichtsteil Tamsulosin-hydrochlorid, liegt.

7. Granulat nach Anspruch 4, welches mit einem Überzugsmaterial überzogen ist.

8. Granulat nach Anspruch 7, wobei das Überzugsmaterial ein polymeres oder magensaftresistentes Überzugsmaterial ist.

9. Granulat nach Anspruch 7, wobei die Menge des Überzugsmaterials im Bereich von 0,2 bis 100 Gewichtsteilen, bezogen auf 1 Gewichtsteil Tamsulosin-hydrochlorid, liegt.

10. Zusammensetzung nach Anspruch 1 und Granulat nach Anspruch 4, wobei die Menge an Polyvinylacetat im Bereich von 40 bis 600, vorzugsweise von 50 bis 300 Gewichtsteilen, bezogen auf 1 Gewichtsteil Tamsulosin-hydrochlorid, liegt, vorzugsweise.

## Revendications

1. Composition pour l'administration par voie orale de chlorhydrate de tamsulosine comprenant du chlorhydrate de tamsulosine, du poly(acétate de vinyle), et de l'hydroxypropylméthylcellulose soluble dans l'eau, où la quantité de poly(acétate de vinyle) s'étend de 20 à 1 000 parties en poids sur la base de 1 partie en poids de chlorhydrate de tamsulosine.

2. Composition selon la revendication 1, où le poly(acétate de vinyle) se présente sous la forme d'une poudre ou d'une suspension comprenant du poly(acétate de vinyle) et un additif pharmaceutiquement acceptable.

3. Composition selon la revendication 1, où la quantité d'hydroxypropylméthylcellulose soluble dans l'eau s'étend de 0,1 à 500 parties en poids sur la base de 1 partie en poids de chlorhydrate de tamsulosine.

4. Granule à libération prolongée de chlorhydrate de tamsulosine comprenant du chlorhydrate de tamsulosine, du poly(acétate de vinyle), une hydroxypropylméthylcellulose soluble dans l'eau, et un agent de granulation, où la quantité de poly(acétate de vinyle) s'étend de 20 à 1 000 parties en poids sur la base de 1 partie en poids de chlorhydrate de tamsulosine.

5. Granule selon la revendication 4, où l'agent de granulation est sélectionné dans le groupe constitué du lactose, de la cellulose microcristalline, du phosphate de calcium dibasique, du phosphate de calcium dibasique dihydraté, du phosphate de calcium tribasique et de leur mélange.

6. Granule selon la revendication 4, où la quantité d'agent de granulation s'étend de 1 à 2 000 parties en poids sur la base de 1 partie en poids de chlorhydrate de tamsulosine.

7. Granule selon la revendication 4, qui est enrobée d'un matériau d'enrobage.

8. Granule selon la revendication 7, où le matériau d'enrobage est un matériau d'enrobage polymère ou entérique.

9. Granule selon la revendication 7, où la quantité du matériau d'enrobage s'étend de 0,2 à 100 parties en poids sur la base de 1 partie en poids de chlorhydrate de tamsulosine.

10. Composition selon la revendication 1 et granule selon la revendication 4, où la quantité de poly(acétate de vinyle) s'étend de 40 à 600, de préférence de 50 à 300 parties en poids sur la base de 1 partie en poids de chlorhydrate de tamsulosine, de préférence.
